# FASCICULE DE BREVET EUROPEEN

(11) **EP 0 465 383 B1**
(45) Date de publication et mention de la délivrance du brevet: **10.09.1997**
(21) Numéro de dépôt: 91420208.0
(22) Date de dépôt: 21.06.1991
(51) Int. Cl.: A61F 2/24

(54) **Valve cardiaque artificielle**
Künstliche Herzklappe
Prosthetic heart valve

(30) Priorité: 22.06.1990 FR 9008191
(43) Date de publication de la demande: 08.01.1992
(73) Titulaire: FABRIQUE D'IMPLANTS ET D'INSTRUMENTS CHIRURGICAUX SOCIETE A RESPONSABILITE LIMITEE, F-43240 Saint Just Malmont (FR)
(72) Inventeur: Baudet, Eugène, Les deux Poteaux F-33700 Merignac (FR); Cuilleron, Jean, F-42100 Saint-Etienne (FR)
(74) Mandataire: Dupuis, François

(56) Documents cités:
- EP-A- 113 681
- EP-A- 211 576
- EP-A- 0 338 179
- US-A- 4 276 658

## Description

Pour certaines lésions valvulaires dont les causes peuvent être diverses, il est parfois nécessaire d'implanter des valves cardiaques artificielles dont la structure mécanique permet de remplacer un appareil valvulaire mitral ou aortique.

Compte tenu des fonctions que doit remplir une valve cardiaque artificielle, dans la mesure du possible, il est important que cette dernière puisse répondre à plusieurs critères :
- La valve doit assurer sa fonction pendant une durée supérieure à l'espoir de vie du malade quel que soit son age au moment où la valve a été implantée. Les caractéristiques de la valve doivent donc être déterminées en conséquence.
- La valve doit permettre un flux laminaire central sans turbulences d'amont ou d'aval pour répondre à des performances hémodynamiques de manière à ne pas induire, au-travers de l'implant, des gradients transvalvulaires significatifs. C'est notamment le cas pour des valves de petit diamètre.
- Compte tenu de l'existence d'aires de stase résultant du principe même du mécanisme des valves artificielles, des caillots fibrocruoriques peuvent se former au niveau de ce mécanisme en étant susceptibles de se détacher et de migrer dans la circulation systémique. Il peut, en outre, en résulter une thrombose responsable du blocage du mécanisme de la valve.

Pour diminuer ce phénomène de thrombogénicité, il est généralement fait appel à un traitement anti-coagulant administré par voie orale. Ce traitement a pour but de limiter la formation des caillots au niveau du mécanisme de la valve.

On a proposé à ce jour plusieurs types de valves de différentes conceptions technologiques déterminées pour tenter de se rapprocher le plus possible des buts fixés rappelés ci-dessus.

On connaît par exemple les valves dont le mécanisme comprend pour l'essentiel une bille à mouvement libre dans une cage métallique présentant des arceaux solidaires d'un anneau métallique. L'existence de la bille au milieu du flot sanguin, peut être responsable d'un flux essentiellement latéral, de sorte que les performances hémodynamiques ne sont pas satisfaisantes.

Pour améliorer ces performances hémodynamiques, on a proposé une valve à disque basculant en créant un flux latéral et l'existence d'une aire principale et d'une aire secondaire. Il est apparu cependant que cette aire secondaire est responsable de stase entraînent de ce fait des phénomènes de thrombose même en cas de traitement anti-coagulant.

Pour tenter de remédier à ces inconvénients et de résoudre le problème de l'hémodynamisme et de la thrombogénicité, on a proposé des valves cardiaques artificielles présentant deux ailettes articulées à l'intérieur d'un anneau. On peut citer par exemple la valve connue sous le nom de valve "ST JUDE MEDICAL" présentant deux ailettes planes équipées d'articulation incluses dans l'anneau de la prothèse et permettant une ouverture desdites ailettes de l'ordre de 85 degrés. Il en résulte un flot quasiment central et laminaire. En outre, avec ce type de valve, le plan des ailettes en position ouverte dépasse le plan de l'anneau d'une valeur réduite, évitant ainsi, notamment en position mitrale, de gêner le mouvement d'ouverture et de fermeture des ailettes par des structures anatomiques sous-jacentes.

Il apparaît cependant qu'en position d'ouverture des ailettes, la valve ne dispose pas de trois aires équivalentes. Les deux aires latérales représentent chacune sensiblement 40 pour cent, tandis que l'aire centrale représente seulement 20 pour cent. Il apparaît donc que l'aire centrale est moins utilisée que les aires latérales, de sorte que les phénomènes de stase à ce niveau, avec un balayage sanguin insuffisant, peuvent générer un phénomène de thrombose nécessitant un traitement anti-coagulant.

On connaît également d'autres types de valves à ailettes incurvées qui présentent, à l'opposé de celles décrites ci-dessus, trois aires équivalentes en position d'ouverture des ailettes. Des essais cliniques ont démontré que l'adoption de trois aires équivalentes, limite d'une manière importante les phénomènes de thromboses valvulaires. Par contre, si l'on peut considérer que le problème de la thrombogénicité est résolu, il n'en est pas de même en ce qui concerne le problème de l'hémodynamisme. En effet, l'ouverture des ailettes est limité à environ 76 degrés engendrant de ce fait, pour les aires latérales, des flots obliques. Un autre inconvénient apparaît également au niveau de la conception technologique en tant que telle de la valve, de sorte qu'en position ouverte, les ailettes sont très en dehors de l'anneau ce qui peut engendrer, notamment en position mitrale, des incidents de blocage desdites ailettes par des structures ventriculaires sous-jacentes.

Cet état de la technique peut être illustré par l'enseignement du Brevet EP 0338179. Outre le fait qu'en position d'ouverture des ailettes, ces dernières sont très en dehors du plan de l'anneau, d'autres inconvénients et problèmes apparaissent compte tenu de la structure de la valve.

Un premier inconvénient se trouve au niveau de l'articulation des ailettes dans l'anneau qui ne délimite pas, entre les extrémités des ailettes une surface totalement lisse mais des empreintes en creux profilées susceptibles de perturber le flot central laminaire.

Un autre inconvénient apparaît également au moment de la fermeture des ailettes qui viennent sur l'anneau, ce qui génère un bruit répétitif audible pour le patient et son entourage.

Enfin, on souligne, compte tenu de la nature du matériau constituant les ailettes, ces dernières sont relativement épaisses, de sorte que leur élasticité n'est pas suffisante pour absorber l'énergie cinétique emmagasinée par la vitesse du flux sanguin.

L'invention s'est donc fixée pour but de remédier à ces inconvénients et de résoudre le problème posé de concevoir une valve cardiaque artificielle dont les caractéristiques sont déterminées pour résoudre à la fois le problème de l'hémodynamique et celui de la thrombogénicité, ainsi que le problème important du bruit au moment de la fermeture des ailettes.

Pour atteindre ces objectifs, d'une manière globale, il a été conçu une valve cardiaque du type de celle définie dans la Revendication 1

Compte tenu de la nature des matériaux pour la réalisation des ailettes notamment, il est possible de les réaliser dans des épaisseurs extrêmement réduites leur conférant une élasticité contrôlée. Il en résulte qu'au moment de la fermeture des ailettes, l'énergie cinétique emmagasinée par la vitesse du flux sanguin est absorbée par les ailettes créant en amont une pression instantanée et, en aval, un vide partiel d'autant plus faibles que l'élasticité de l'ensemble est importante.

Il apparaît également que la déformation des ailettes associée au frottement de leur extrémité sur le corps forment un amortisseur à la fermeture permettant d'allonger le temps de décélération de l'ensemble en mouvement. Il en résulte que le phénomène du coup de bélier du flux sanguin est fortement diminué, ce qui a pour effet d'atténuer le bruit de fermeture des ailettes et d'augmenter le temps de freinage tout en supprimant tous phénomènes de cavitation en aval des ailettes.

Il apparaît donc que la valve, selon l'invention, a une thrombogénicité minimale autorisant éventuellement, en période post-opératoire, un bas régime d'anticoagulation du fait de la diminution des aires de stase, compte tenu de la réduction du gradient transprothétique, de l'existence d'un flux axial et laminaire également réparti au niveau des trois aires équivalentes, et lavant parfaitement les deux faces des ailettes.

Pour résoudre le problème posé, d'une part, d'assurer le balayage et le nettoyage de la surface interne de l'anneau où sont articulées les ailettes et, d'autre part, d'avoir entre les ailettes, au niveau de leur articulation une surface suffisamment large et totalement libre pour perturber le flux central, l'articulation de ces dernières s'effectue par des parties hémisphériques mâles formées en débordement du profil de chaque ailette et aptes à coopérer avec des cavités hémisphériques complémentaires formées dans les deux oreilles de l'anneau.

D'une manière particulièrement avantageuse, il apparaît que cette conception de l'articulation des ailettes permet de laisser entre les extrémités desdites ailettes un espace entièrement libre exempt de toutes aspérités en creux ou en relief permettanten conséquence un écoulement du flux central dans des conditions optimum.

En outre, il en résulte un lavage des articulations compte tenu d'un léger dégagement ou allégement des parties hémisphériques mâles dans leur cavité complémentaire du au mouvement de flexion alterné des ailettes permettant à cet instant un flux et reflux nettoyant lesdites cavités.

Les parties mâles hémisphériques peuvent se présenter sous une forme trifoliée comportant trois évidements décalés de 60 degrés.

L'invention est exposée ci-après plus en détail à l'aide des dessins annexés dans lesquels :
La figure 1 est une vue en perspective de la valve en position d'ouverture des ailettes selon l'invention.
La figure 2 est, à grande échelle, une vue de face avec coupe partielle de la valve en position d'ouverture des ailettes.
La figure 3 est une vue en coupe transversale considérée selon la ligne 3-3 de la figure 2.
La figure 4 est une vue semblable à la figure 2 en position de fermeture des ailettes.
La figure 5 est une vue en coupe transversale considérée selon la ligne 5-5 de la figure 4.
La figure 6 montre l'implantation de la valve en position mitrale.
La figure 7 montre l'implantation de la valve en position aortique.
La figure 8 montre l'implantation de la valve en position double, c'est-à-dire,en position mitrale et aortique.

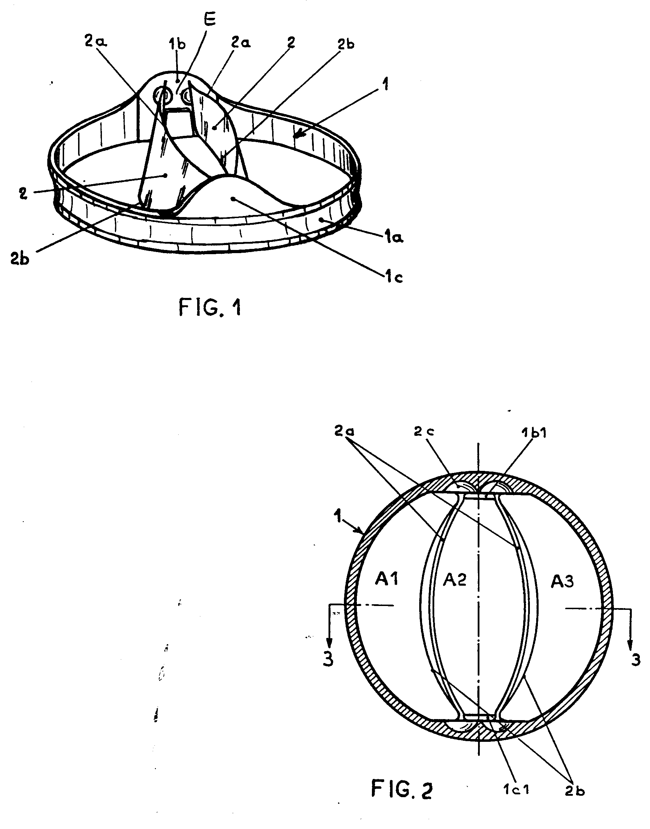

Comme le montre la figure 1, la valve artificielle comprend un anneau (1) à l'intérieur duquel sont articulées deux ailettes identiques (2). L'anneau (1) présente tout agencement extérieur permettant d'une manière connue l'insertion d'une collerette de tissu pour constituer l'anneau de suture en tant que tel. Par exemple ces agencements sont constitués par une gorge (1a).

On rappelle que la valve est destinée aussi bien au remplacement de la valvule mitrale qu'à celui de la valvule aortique sans autre modification que celle de l'anneau de suture dont la forme est différente selon la position envisagée : aortique ou mitrale.

Suivant l'invention, l'anneau (1) présente des agencements internes aptes à assurer l'articulation des ailettes (2) selon une position d'ouverture maximale pour délimiter, en combinaison avec le profil des ailettes, trois aires équivalentes (A1, A2, A3) permettant un flux central, axial et laminaire. En outre, selon une autre caractéristique, et comme il sera indiqué dans la suite de la description, les agencements internes de l'anneau permettent, en position de fermeture des ailettes (2), leur intégration à l'intérieur de l'anneau.

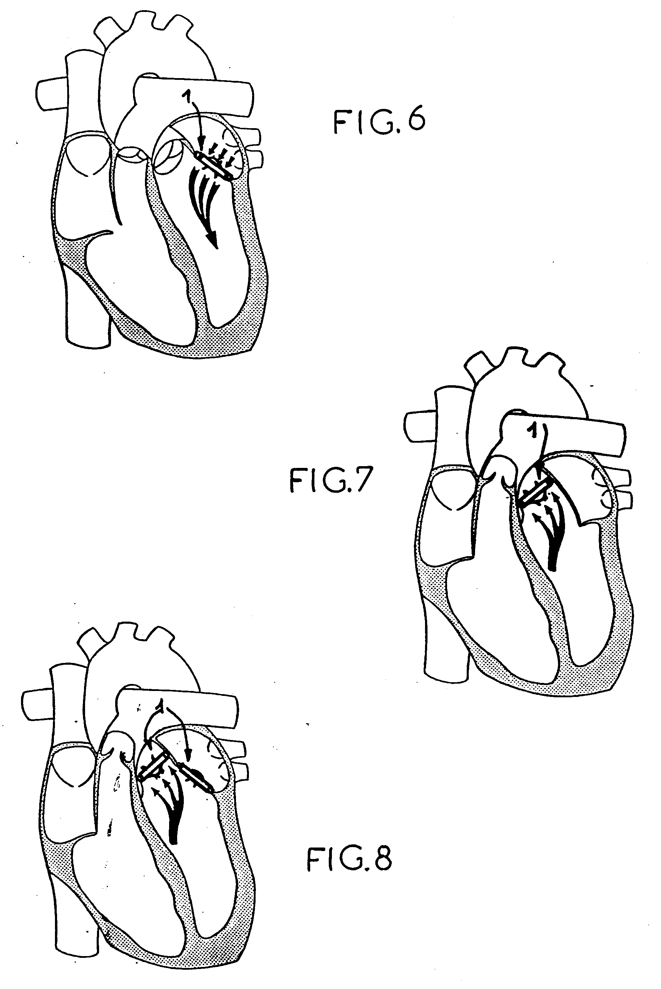

Comme le montrent notamment les figures 1, 3 et 5, les agencements internes de l'anneau (1) sont constitués par deux parties diamétralement opposées (1b et 1c) formées directement en débordement de l'une des faces de l' anneau en constituant des épaulements ou oreilles. Chacune des parties (1b, 1c) apparaît du côté de la face d'admission de la valve. Les deux parties proéminentes (1b, 1c) recevant le mécanisme d'articulation des ailettes (2) et constituant des épaulements ou oreilles, assurent le déport du mécanisme de telle façon que les ailettes (2) en position ouverte ne dépassent le plan de l'anneau que d'une valeur réduite variant selon leur taille.

Suivant une autre caractéristique, chacune des parties (1b, 1c) constitue, sur la totalité de la largeur de l'anneau, une surface interne méplate présentant des moyens en relief (1b1, 1c1) conformés pour assurer le blocage angulaire des ailettes selon un angle d'au moins 85 degrés.

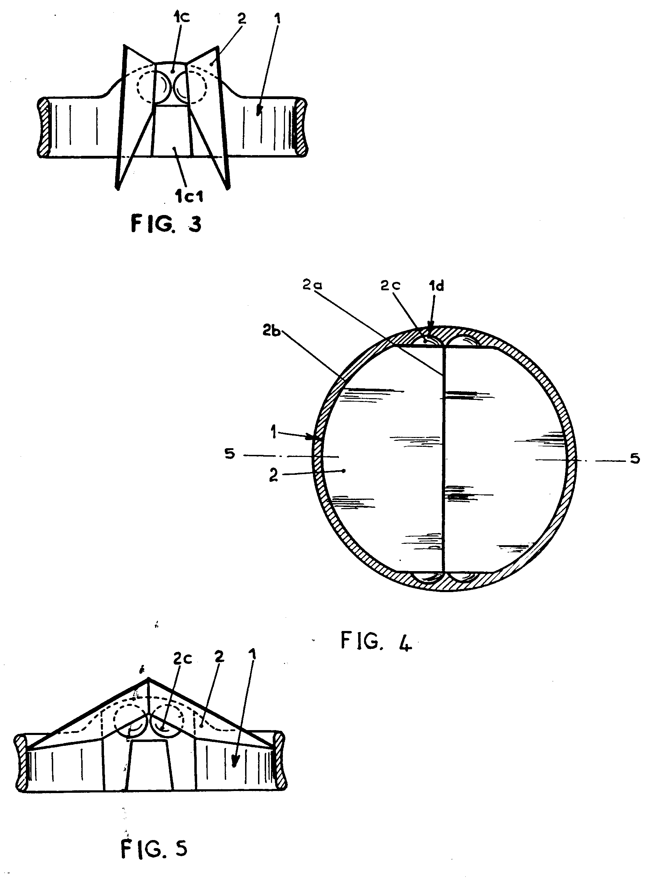

Dans ce but, ces moyens (1b1, 1c1) sont constitués chacun par un bossage formé en débordement de la face méplate interne de chacune des parties (1b, 1c) à l'opposé de l'épaulement recevant le mécanisme d'articulation des ailettes. Les bords latéraux de chacun des bossages (1b1, 1c1) sont inclinés selon un angle approprié pour permettre, comme indiqué, le blocage en position d'ouverture de chacune des ailettes à au moins 85 degrés. En outre, cet angle est déterminé pour qu'en position d'ouverture des ailettes, ces dernières aient tendance à converger en direction de chacun des épaulements (1b, 1c) recevant le mécanisme d'articulation (figure 3).

Suivant une autre caractéristique, chacune des ailettes a un profil en section incurvé de telle façon qu'en position ouverte, l'orifice central délimité par les ailettes présente une aire centrale (A2) équivalente ou supérieure à chacune des deux aires latérales (A1 et A3). Cette position d'ouverture limitée à au moins 85 degrés permet d'assurer un flot axial et laminaire.

Les bords opposés (2b) de chacune des ailettes (2) sont profilés suivant une courbure apte à épouser le rayon interne de l'anneau. Les bords (2a) présentent une courbure appropriée et déterminée pour assurer un contact jointif entre les deux ailettes. En position de fermeture, les ailettes forment un angle obtus.

L'articulation de chacune des ailettes (2), au niveau de la section méplate des épaulements (1b, 1c), s'effectue par des parties hémisphériques mâles (2c) formées à chaque extrémité directement dans le prolongement des bords de chaque ailette. Chacune de ces parties hémisphériques (2c) est apte à coopérer avec des cavités hémisphériques complémentaires (1d) formées au niveau de la section méplate de chaque épaulement. Ces cavités (1d) ne sont séparées que de quelques millimètres. A noter que la partie mâle hémisphérique solidaire de l'ailette peut constituer soit une hémisphère totale, soit une hémisphère trifoliée entaillée de trois évidements décalés angulairement selon un angle de 60 degrés environ. De telles dispositions permettent d'assurer à chacun des mouvements d'articulation un balayage de la surface concave visant à limiter la formation de thromboses au niveau de l'articulation.

D'une manière particulièrement importante et comme il ressort des figures 1, 2 et 4 notamment des dessins, l'intégration des parties d'articulation (2c) dans les cavités complémentairse (1d) laisse subsister un espace (E) suffisamment important entre les extrémités des ailettes. Cet espace libre (E) ne présente aucune aspérité en creux ou en relief susceptible de constituer un obstacle à l'écoulement du flux laminaire central.

Quel que soit son mode de réalisation, compte tenu du principe du mécanisme défini ci-dessus, ce dernier n'offre aucun point de stase au flot sanguin. Les seules structures fixes dans le champ d'action de la valve résultent de l'épaisseur des ailettes qui peut être extrêmement réduite compte tenu de la nature du matériau employé.

Suivant une autre caractéristique importante de l'invention, l'anneau (1) et les ailettes (2) sont réalisés en titane ou alliage de titane recouvert d'une pellicule de carbone amorphe et isotrope. La résistance élevée de cet alliage de titane, permet d'avoir des ailettes et un anneau d'apaisseur très réduite, tout en augmentant considérablement les caractéristiques mécaniques de la valve. Par ailleurs, la pellicule de recouvrement du titane sous forme d'une couche mince de carbone amorphe, constitue une structure continue d'un aspect totalement lisse non poreux et avec une moindre tension superficielle. Il en résulte une mouillabilité très faible assurant un écoulement du sang sans ralentissement et lui conférant ainsi de très bonnes performances hémodynamiques.

Les avantages resssortent bien de la description. En particulier, on souligne et on rappelle :
- L'orifice géométrique utile supérieur à celui des valves en carbone pyrolitique, d'un diamètre extérieur équivalent, compte tenu, d'une part, de la faible épaisseur de l'anneau qui permet une section de passage plus importante et, d'autre part, de la faible épaisseur des ailettes exécutées ainsi que ledit anneau en titane ou alliage de titane.
- L'ouverture des ailettes selon un angle d'au moins 85 degrés ménagent trois aires équivalentes autorisant un flux central axial et laminaire.
- L'intégration du mécanisme de l'articulation dans l'anneau réduit de ce fait les structures fixes responsables d'aires de stase.
- Le bas profil de la valve permet aux ailettes de ne pas être gênées par une interférence avec des éléments anatomiques sous-jacents étant donné que les ailettes en position d'ouverture débordent du plan de l'anneau d'une valeur extrêmement réduite quasiment nulle.
- L'application du matériau substrat titane-carbone amorphe autorise des épaisseurs plus faibles pour les parties mobiles, diminuant leur masse donc leur inertie, permettant d'accroître la section de passage du flux sanguin dans des proportions significatives, ce qui devrait réduire les pertes de charges transvalvulaires
- La déformation des ailettes, compte tenu de la nature des matériaux les composant, fait office d'amortisseur à la fermeture permettant, d'une part, d'allonger le temps de décélération et, d'autre part, d'atténuer le bruit de fermeture.
- L'espace important et entièrement libre qui apparaît entre les extrémités des ailettes au niveau de leur articulation.

## Revendications

1. Valve cardiaque artificielle comprenant un anneau (1) à l'intérieur duquel sont articulées deux ailettes courbes (2) s'ouvrant au moins à 85° et délimitant, en position ouverte, trois aires hydrauliquement équivalentes (A1) (A2) (A3) dans le plan de l'anneau (1), caractérisée par la combinaison des dispositions suivantes :
- les ailettes (2) sont recouvertes d'une pellicule de carbone amorphe et isotrope, pour avoir une limite d'élasticité élevée, une ductibilité et une résilience importantes afin de conférer aux dites ailettes (2) une épaisseur réduite et une élasticité contrôlée,
- les axes d'articulation (2c) des ailettes (2) sont disposés au dessus et en dehors du plan médian de l'anneau (1) pour réduire le profil de la valve lorsque lesdites ailettes (2) sont en position d'ouverture,
- les extrémités des axes d'articulation (2c) des ailettes (2) présentent des agencements aptes à coopérer avec des agencements complémentaires (1d) formés dans l'épaisseur d'une partie déportée au dessus et en dehors du plan de l'anneau, lesdits agencements (1d) étant aptes à permettre un mouvement de rotation, sans mouvement de translation,
- les bords externes (2b) des ailettes (2) sont profilés sous forme d'un arc partiellement élliptique pour épouser parfaitement le profil interne de l'anneau (1) au dessus du plan médian de l'anneau lorsqu'elles sont en position de fermeture,
- les profils (2a) (2b) des bords internes et externes des ailettes (2) sont déterminés, pour permettre, avec le rayon de courbure de la partie médiane des ailettes, de délimiter les trois aires hydrauliquement équivalentes (A1) (A2) (A3) dans le plan de l'anneau, de dégager le profil des ailettes (2) au droit des agencements d'articulation (2c) (1d) et d'assurer une meilleure pénétration dans le flux sanguin, et
- la partie déportée au dessus et en dehors du plan de l'anneau (1) apte à recevoir les axes d'articulation (2c) des ailettes (2), est formée par deux projections diamétralement opposées (1b) (1c) , s'étendant de la base de l'anneau vers la face d'admission du flux sanguin, chaque projection présente une surface externe cylindrique et une surface interne méplate, intégrant des reliefs (1b1) (1c1) conformés pour assurer le blocage des ailettes (2), en position d'ouverture au moins à 85°.

2. Valve selon la revendication 1, caractérisée en ce que les agencements des axes d'articulation (2c) sont constitués par des parties hémisphériques mâles aptes à coopérer avec des cavités hémisphériques complémentaires (1d) formées au niveau de la section méplate interne de chaque projection (1b) (1c).

3. Valve selon la revendication 2, caractérisée en ce que les parties mâles hémisphériques (2c) sont trifoliées et peuvent présenter des évidements décalés angulairement, notamment trois évidements décalés de 60 degrés.

## Claims

1. Artificial heart valve comprising a ring (1) which encloses two hinged curved wings (2) which open to at least 85° and, in the opened position, delimit three hydraulically equivalent areas (A1) (A2) (A3) in the plane of ring (1) characterised by a combination of the following arrangements:
- the wings (2) are covered in a film of isotropic amorphous carbon in order to achieve a high elastic limit as well as good ductility and strength so that said wings (2) are thin and of controlled elasticity,
- the hinge pins (2c) of the wings (2) are arranged above and outside the median plane of ring (1) in order to reduce the profile of the valve when said wings (2) are in the opened position,
- the ends of the hinge pins (2c) of wings (2) have features capable of cooperating with matching features (1d) formed in the thickness of one part that is offset above and outside the plane of the ring, said features (1d) being capable of allowing a movement of rotation without any movement of translation,
- the external edges (2b) of wings (2) are formed in the shape of a partially elliptical arc so that they perfectly match the internal profile of ring (1) above the median plane of the ring when they are in the closed position,
- the profiles (2a) (2b) of the inside and outside edges of wings (2) are designed so as to make it possible, in conjunction with the radius of curvature of the median part of the wings, to delimit the three hydraulically equivalent areas (A1) (A2) (A3) in the plane of the ring, to release the profile of the wings (2) at the level of the hinge features (2c) (1d) and to ensure improved penetration into the blood flow, and
- the part that is offset above and outside the plane of ring (1) capable of accommodating the hinge pins (2c) of wings (2) is formed by two diametrically opposite projections (1b) (1c) extending from the base of the ring towards the blood-flow inlet surface, each projection having a cylindrical external surface and a flat internal surface that include bulges (1b1) (1c1) shaped to ensure wings (2) are locked in an opened position of at least 85°.

2. Valve as claimed in claim 1 characterised in that the features of the hinge pins (2c) consist of male hemispherical parts capable of cooperating with matching hemispherical cavities (1d) formed at the level of the flat internal section of each projection (1b) (1c).

3. Valve as claimed in claim 2 characterised in that the male hemispherical parts (2c) are trifoliate and may have angularly offset recesses, especially three recesses offset at 60 degrees.

## Patentansprüche

1. Künstliche Herzklappe mit einem Ring (1), innerhalb dessen zwei gebogene Flügel (2) gelenkig gelagert sind, die sich mindestens um 85° öffnen und in geöffneter Stellung in der Ringebene (1) drei hydraulisch gleichwertige Flächen (A1) (A2) (A3) umschreiben, gekennzeichnet durch die Kombination der folgenden Vorrichtungen:
- die Flügel (2) sind mit einem Film aus amorphem und isotropem Kohlenstoff überzogen, um eine hohe Elastizitätsgrenze sowie eine große Duktilität und Schlagzähigkeit zu erzielen, damit die besagten Flügeln (2) schmaler ausfallen können und ihnen eine kontrollierte Elastizität verliehen wird,
- die Gelenkachsen (2c) der Flügel (2) sind oberhalb und außerhalb der mittleren Ringebene (1) angeordnet, um das Klappenprofil zu verringern, wenn sich die Flügel (2) in geöffneter Stellung befinden,
- die Enden der Gelenkachsen (2c) der Flügel (2) weisen Vorkehrungen auf, die geeignet sind, mit ergänzenden, in der Materialdicke ausgebildeten Vorkehrungen (1d) eines oberhalb und außerhalb der Ringebene versetzten Teils zusammenzuwirken, wobei diese Vorkehrungen (1d) geeignet sind, eine translationsfreie Drehbewegung zu ermöglichen,
- die Außenränder (2b) der Flügel (2) besitzen das Profil eines teilweise elliptischen Bogens, um das Innenprofil des Ringes (1) oberhalb der mittleren Ebene des Ringes bei geschlossener Flügelstellung vollkommen nachzubilden,
- die Profile (2a) (2b) der Innen- und Außenränder der Flügel (2) sind so beschaffen, um in der Ringebene zusammen mit dem Krümmungsradius des mittleren Teils der Flügel drei hydraulisch gleichwertige Flächen (A1) (A2) (A3) zu umschreiben, das Profil der Flügel (2) im Bereich der Gelenkvorkehrungen (2c) (1d) freizusetzen und ein besseres Eindringen in den Blutstrom zu gewährleisten, und
- der oberhalb und außerhalb der Ringebene (1) versetzte Teil, der geeignet ist, die Gelenkachsen (2c) der Flügel (2) aufzunehmen, wird durch zwei diametral entgegengesetzte Vorsprünge (1b) (1c) gebildet, die sich von der Ringbasis aus bis zur Einlaßseite des Blutstroms erstrecken, wobei jeder Vorsprung eine zylindrische Außenfläche und eine abgeflachte Innenfläche aufweist und Reliefs (1b1) (1c1) besitzt, die so ausgebildet sind, um in einer Öffnungsposition von mindestens 85° die Blockierung der Flügel (2) zu gewährleisten.

2. Klappe nach Anspruch 1, dadurch gekennzeichnet, daß die Vorkehrungen der Gelenkachsen (2c) aus halbkugelförmigen männlichen Teilen bestehen, die geeignet sind, mit ergänzenden halbkugelförmigen Vertiefungen (1d) im Bereich des abgeflachten Innenabschnitts des jeweiligen Vorsprungs (1b) (1c) zusammenzuwirken.

3. Klappe nach Anspruch 2, dadurch gekennzeichnet, daß die halbkugelförmigen männlichen Teile (2c) dreiblättrig sind und winkelig versetzte Aussparungen - vor allem drei um 60 Grad versetzte Aussparungen - aufweisen können.
